# NEW EUROPEAN PATENT SPECIFICATION

(11) **EP 1 169 314 B2**
(45) Date of publication and mention of the opposition decision: **01.06.2011**
(45) Mention of the grant of the patent: 04.09.2002
(21) Application number: 01909568.6
(22) Date of filing: 28.02.2001
(51) Int. Cl.: C07D 307/87

(54) **PROCESS FOR THE MANUFACTURE OF SALTS OF CITALOPRAM**
VERFAHREN ZUR HERSTELLUNG VON CITALOPRAM-SALZEN
PROCÉDÉ DE PRÉPARATION DE SELS DE CITALOPRAM

(30) Priority: 13.03.2000 DK 200000402 U; 13.04.2000 WO PCT/DK00/00183
(43) Date of publication of application: 09.01.2002
(60) Divisional application: 02009350.6
(73) Proprietor: H. Lundbeck A/S, 2500 Valby-Copenhagen (DK)
(72) Inventor: PETERSEN, Hans, 2500 Vanlöse (DK); BÖGESÖ, Klaus, Peter, 2970 Hörsholm (DK); HOLM, Per, 2720 Vanlöse (DK)
(74) Representative: HOFFMANN EITLE
(86) International application number: PCT/DK2001/000137
(87) International publication number: WO 2001/068627

(56) References cited:
- EP-A- 1 125 907
- EP-A1- 0 347 066
- WO-A-00/23431
- WO-A-98/19511
- WO-A-98/19512
- WO-A-98/19513
- WO-A2-00/11926
- DE-A1- 2 657 013
- K. A. Jorgenson, Expert Declaration (03.05.2002)
- H. Petersen, Citalopram Crystallisation Experiments (06.05.2002)
- T. Laird, Statement about Industrial purification methods (06.03.2002)

## Description

The present invention relates to a process for the preparation of purified salts of the well known antidepressant drug citalopram,1-[3-(dimethylamino)propyl]-1-(4-fluorophenyl)-1,3-dihydro-5-isobenzofurancarbonitrile.

### Background of the Invention

Citalopram is a well-known antidepressant drug that has now been on the market for some years and has the following structure:

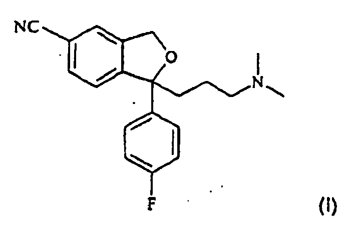

It is a selective, centrally acting serotonin (5-hydroxytryptamine; 5-HT) reuptake inhibitor, accordingly having antidepressant activities. The antidepressant activity of the compound has been reported in several publications, eg. J. Hyttel, Frog. Neuro-Psychopharmacol. & Biol. Psychiat., 1982, 6, 277-295 and A. Gravem. Acta Psychiatr. Scand., 1987, 75, 478-486. The compound has further been disclosed to show effects in the treatment of dementia and cerebrovascular disorders, EP-A-474580.

Citalopram was first disclosed in DE 2,657,013, corresponding to US 4,136,193. This patent publication describes the preparation of citalopram by one method and outlines a further method, which may be used for preparing citalopram. The citalopram prepared was isolated as the oxalate, the hydrobromide and the hydrochloride salt, respectively. Furthermore, the citalopram base was obtained as an oil (B.P. 175 C/0.03 mmHg). Citalopram is marketed as the hydrobromide and the hydrochloride, respectively.

A number of processes for the preparation of citalopram have been disclosed. In many of these, the last step of the process is a conversion of a group different from cyano in the 5 position of the direct analogue of citalopram to a 5-cyano group. So citalopram has been prepared by:
Exchange of 5-halogen, or 5-CF₃-(CF₂)ₙ-SO₂-O- with cyano (DE 2,657,013 and co-pending WO 0011926 and WO 0013648)
Conversion of a 5-amido or 5-ester group to a 5-cyano group (WO 9819513)
Conversion of a 5-amino group to a 5-cyano group (WO 9819512)
Conversion of a 5-formyl group to a 5-cyano group (WO 9930548)
Conversion of a 5-oxazolinyl or 5-thiazolinyl group to a 5-cyano group (WO 0023431)

Other processes for the preparation of citalopram comprise exchange of the 5-bromo group of 1-(4-fluorophenyl)-1,3-dihydro-5-isobenzofuranbromide with 5-cyano followed by alkylation with a 3-(N,N-dimethylamino)propyl-halogenide (DE 2,657,013 and WO 9819511).

Many of the processes mentioned above have the disadvantage that it is difficult to separate the intermediates formed during the process (the intermediates mentioned above or earlier intermediates) from the end product and, accordingly, extensive purification procedures involving loss of citalopram are required in order to obtain the necessary quality of the end product.

It has now been found that the base of citalopram may be obtained as a very nice and pure crystalline product, which may easily be handled and conveniently be formulated into tablets and other pharmaceutical forms. Furthermore, it has surprisingly been found that a very good and efficient purification of citalopram maybe obtained during manufacture of citalopram (e.g. of the hydrobromide or the hydrochloride salt) by crystallising the base, and thereafter forming a salt from the base.

This purification process is particularly useful for removing intermediates which are structurally closely related to citalopram, in particular compounds which only differ from citalopram by the substituent situated in position 5 on the isobenzofurane ring, and intermediates which have physical/chemical properties which are close to those of citalopram, e.g. the 1-[3-(dimethylamino)propyl]-1-(4-fluorophenyl)-1,3-dihydro-isobenzofuranes having halogen (in particular bromide and chloride), an amide or an ester in position 5 of the isobenzofurane ring, or 1-(4-fluorophenyl)-1,3-dihydro-5-isobenzofuranbromide, or -cloride.

### Summary of the invention

The present invention relates to a process for the manufacture of a salt of citalopram characterised in that one or more impurities of the formula 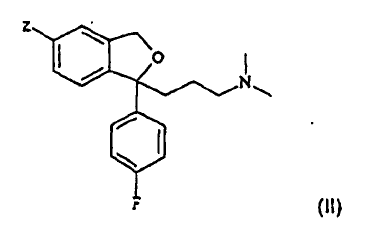 wherein Z is halogen are removed from a crude mixture of citalopram or from a crude salt of citalopram, by precipitating citalopram base in crystalline form and transferring said base into a salt thereof.

The crude mixture of citalopram containing the compound of formula II as an impurity may be prepared by subjecting a compound of formula II to a cyanide cxchange reaction with a cyanide source, or by subjecting 1-(4-fluorophenyl)-1,3-dihydro-5-isobenzofuranhalogenide, in particular the bromide, to a cyanide exchange reaction followed by alkylation with a 3-(N,N-dimethylamino)propyl-halogenide.

In a particular embodiment of the invention, Z is bromide or chloride.

In a particularly preferred embodiment of the invention, the salt prepared is the hydrobromide or hydrochloride salt of citalopram.

The crude salt may be any convenient salt, such as the hydrobromide, hydrochloride, sulphate, oxalate, phosphate, nitrate or any other convenient salt. Other salts are salts of organic acids.

In a preferred embodiment of the invention, the crude salt is the sulphate, the hydrobromide or the hydrochloride ride salt.

A hydrobromide or hydrochloride prepared from the free base of citalopram can be used in a pharmaceutical formulation. Preferably the formulation is for oral administration.

The formulations may be prepared by direct compression of citalopram in admixture with conventional adjuvants or diluents. Alternatively, a wet granulate or a melt granulate of citalopram, optionally in admixture with conventional adjuvants or diluents may be used for compression of tablets.

In particular, the pharmaceutical composition contains the racemic mixture of citalopram base, citalopram hydrochloride or citalopram hydrobromide.

The crystalline base of citalopram is preferably more than 99.8% w/w pure, most preferably more than 99.9% w/w pure (peak area). The melting point is preferably a range within 90 - 93 °C, most preferably 91 - 92 °C (DSC; onset, open capsule) or it is between 92 and 94°C, preferably 92.5 and 93.5°C (DSC; onset, closed capsule). The crystalline base of citalopram is preferably in racemic form.

The terms "crude salt" and "crude mixture" refer to the fact that the salt and the mixture, respectively, comprise impurities, in particular impurities of formula II, which must be removed or which it is desired to remove.

The crude salt may be a salt separated directly from the reaction mixture, or the crude reaction mixture may have been subjected to some initial purification, e.g. one re-crystallisation, and /or treatment with activated carbon or silica gel, and the salt formed subsequently by treatment with an acid using methods known in the art. The salt may be isolated by precipitation or it may exist in a solvent, e.g. in the mixture resulting directly from the synthesis of the salt.

Similarly, the crude mixture comprising citalopram may be obtained directly from the synthesis of the compound according to any of the above mentioned processes or it may have been subjected to some initial or simultaneous purification, e.g. one re-crystallisation, treatment with activated carbon or silica gel.

The base of citalopram may be set free from the crude salt by dissolving the crude salt in a mixture of water and an organic solvent and then adding a base. The organic solvent may be toluene, ethyl acetate or any other suitable solvent and the base may be any convenient base, preferably NaOH or NH₃. Likewise, the base of citalopram may, if necessary, be set free from a crude mixture containing citalopram by treatment with a base.

Crude mixtures containing citalopram base may be subjected to further purification and extraction, before the base is precipitated in crystalline form. The base of citalopram may be isolated by separation of the organic phase, evaporation of the solvent in order to obtain the base most probably as an oil and then crystallisation of the base from an aprotic solvent, such as an alkane, including n-beptane, hexane and isooctane, and high and low boiling petroleum ethers and substituted aromates, incl toluene and xylenes.

The pharmaceutically acceptable salt of citalopram, such as the hydrobromide or hydrochloride, may be prepared by methods known in the art. So, the base may be reacted with either the calculated amount of acid in a water miscible solvent, such as acetone or ethanol, with subsequent isolation of the salt by concentration and cooling, or with an excess of the acid in a water immiscible solvent, such as ethylether, ethylacetate or dichloromethane, with the salt separating spontaneously. The hydrobromide or hydrochloride of citalopram obtained by the method of the invention has a very high purity, preferably more than 99,8% pure, most preferably more than 99,9 % purity. Other salts of citalopram, e.g. the oxalate, may also be obtained in a very pure form by this process.

The cyanide exchange reactions mentioned above may be carried out as described in the patent applications mentioned above.

In particular, the conversion to a cyano group may be carried out by reaction with a cyanide source, for example KCN, NaCN, CuCN, Zn(CN)₂ or (R⁴)₄NCN where R⁴ indicates four groups which may be the same or different and are selected from hydrogen and straight chain or branched alkyl, in the presence of a palladium catalyst and a catalytic amount of Cu⁺ or Zn²⁺, or with Zn(CN)₂ in the presence of a palladium catalyst.

The cyanide source is used in a stoichiometric amount or in excess, preferably 1-2 equivalents are used pr. equivalent starting material. R⁴N⁺ may conveniently be (Bu)₄N⁺. The cyanide compound is preferably NaCN or KCN or Zn(CN)₂.

The palladium catalyst may be any suitable Pd(0) or Pd(II) containing catalyst, such as Pd(PPh₃)₄, Pd₂(dba)₃, Pd(PPh)₂Cl₂, etc. The Pd catalyst is conveniently used in an amount of 1-10, preferably 2-6, most preferably about 4-5 mol%.

Catalytic amounts of Cu⁺ and Zn²⁺, respectively, means substoichiometric amounts such as 0.1 - 5, preferably 1 - 3 %. Conveniently, about ½ eq. is used per eq. Pd. Any convenient source of Cu⁺ and Zn⁺⁺ may be used. Cu⁺ is preferably used in the form of CuI and Zn²⁺ is conveniently used as the Zn(CN)₂ salt.

When Z is Br or I, the conversion to a cyano group may also be carried out by reaction with Cu(CN) without catalyst. In a preferred embodiment, the reaction is performed at elevated temperature.

In another aspect of the invention, the reaction is performed in an ionic liquid of the general formula (R⁵)₄N⁺, X, wherein R⁵ are alkyl-groups or two of the R⁵ groups together form a ring and X⁻ is the counterion. In one embodiment of the invention, (R⁵)₄N⁺X⁻ represents

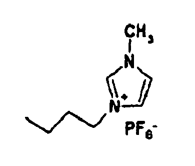

In another particular aspect, the reaction is conducted with apolar solvents such as benzene, xylene or mesitylene and under the influence *of* microwaves by using *i.e.* Synthewave 1000™ by Prolabo. In a particular aspect, the reaction is performed without added solvent.

The temperature ranges are dependent upon the reaction type. If no catalyst is present, preferred temperatures are in the range of 100-200°C. However, when the reaction is conducted under the influence of microwaves, the temperature in the reaction mixture may raise to above 300 °C. More preferred temperature ranges are between 120-170°C. The most preferred range is 130-150°C. If catalyst is present, the preferred temperature range is between 0 and 100°C. More preferred are temperature ranges of 40-90°C. Most preferred temperature ranges are between 60-90°C.

Other reaction conditions, solvents, etc. arc conventional conditions for such reactions and may easily be determined by a person skilled in the art.

When Z is Cl or Br, the conversion to a cyano group may also be carried out by reaction with a cyanide source, for example KCN, NaCN, CuCN, Zn(CN)₂ or (R⁴)₄NCN where (R⁴)₄ indicates four groups which may be the same or different and are selected from hydrogen and straight chain or branched alkyl, in the presence of a nickel catalyst.

The nickel catalyst may be any suitable Ni(0) or Ni(II) containing complex which acts as a catalyst, such as Ni(PPh₃)₃, (σ-aryl)-Ni(PPh₃)₂Cl, etc. The nickel catalysts and their preparation are described in WO 96/11906, EP-A-613720 or EP-A-384392.

In one embodiment of the invention, the reaction is carried out in the presence of a catalytic amount of Cu⁺ or Zn²⁺.

In a particularly preferred embodiment, a Nickel(0) complex is prepared *in situ* before the cyanation reaction by reduction of a Nickel(II) precursor such as NiCl₂ or NiBr₂ by a metal, such as zinc, magnesium or mangan in the presence of excess of complex ligands, preferably triphenylphosphin.

The Ni-catalyst is conveniently used in an amount of 0.5-10, preferably 2-6, most preferably about 4-5 mol%.

Catalytic amounts of Cu⁺ and Zn²⁺, respectively, mean substoichiometric amounts such as 0.1 - 5, preferably 1 - 3 %. Any convenient source of Cu⁺ and Zn²⁺ may be used. Cu⁺ is preferably used in the form of CuI and Zn²⁺ is conveniently used as the Zn(CN)₂ salt or formed in situ by reduction of a Nickel (II) compounds using zinc.

The Ni catalysts are *i.e.* Ni (0), Pd(0) or Pd(II) catalysts as described by Sakakibara et. al. in Bull. Chem. Soc. Jpn., 61, 1985-1990, (1988). Preferred catalysts are Ni(PPh₃)₃ or Pd(PPh₃)₄, or Pd(PPh)₂Cl₂.

The reactions may be performed in any convenient solvent as described in Sakakibara et. al. in Bull. Chem, Soc. Jpn., 61, 1985-1990, (1988). Preferred solvents are acetonitril, ethylacetat, THF, DMF or NMP.

The compounds of formula (II) may be prepared as described in DE 2,657,013, WO 0011926 and WO 0013648, WO 9819513, WO 9819512 and WO 9930548.

Throughout this specification with claims halogen means chloro, bromo or iodo.

The term alkyl refers to a branched or unbranched alkyl group, such as methyl, ethyl, 1-propyl, 2-propyl, 1-butyl, 2-butyl, 2-methyl-2-propyl, and 2-methyl-1-propyl.

The term aryl refers to a carbocyclic aromatic group, in particular phenyl. Aralkyl refers to an arylalkyl group wherein aryl and alkyl is as defined above. The aryl and aralkyl groups may optionally be substituted, e.g. with alkyl groups, forming for example tolyl.

The pharmaceutical compositions may be administered in any suitable way and in any suitable form, for example orally in the form of tablets, capsules, powders or syrups, or parenterally in the form of usual sterile solutions for injection. Preferably the pharmaceutical compositions are administered orally.

The pharmaceutical formulations may be prepared by conventional methods in the art. For example, tablets may be prepared by mixing the active ingredient with ordinary adjuvants and/or diluents and subsequently compressing the mixture in a conventional tabletting machine. Examples of adjuvants or diluents comprise: Com starch, potato starch, talcum, magnesium stearate, gelatine, lactose, gums, and the like. Any other adjuvant or additive colourings, aroma, preservatives etc. may be used provided that they are compatible with the active ingredients.

In particular, the formulations may be prepared by direct compression of citalopram in admixture with conventional adjuvants or diluents. Alternatively, a wet granulate or a melt granulate of citalopram, optionally in admixture with conventional adjuvants or diluents may be used for compression of tablets.

Solutions for injections may be prepared by solving the active ingredient and possible additives in a part of the solvent for injection, preferably sterile water, adjusting the solution to the desired volume, sterilisation of the solution and filling in suitable ampoules or vials. Any suitable additive conventionally used in the art may be added, such as tonicity agents, preservatives, antioxidants, etc.

According to the present invention, the base of citalopram has been found to be crystalline with stable and nice white crystals and it has been found that the base may easily be crystallised in a very pure form. So for example more than 99.8% w/w pure citalopram base was obtained by crystallisation from up to 95% pure hydrobromide without further purification. Accordingly, the process of the invention for preparing salts of citalopram has been found to give the salts as very pure products of pharmaceutically acceptable quality. Accordingly, the yield may be improved substantially during the manufacture of citalopram.

Finally, it has been found that the crystalline citalopram base may be formulated into very good and stable solid formulations with good release properties.

The invention is further illustrated by the following examples.

### Example 1

### Crystallisation of the intermediate R,S-Citalopram as the free base.

### 1-(3-Dimethylaminopropyl)-1-(4'-fluorophenyl)-1,3-dihydrobenzofuran-5-carbonitrile.

1-(3-Dimethylaminopropyl)-1-(4'-fluorophenyl)-1,3-dihydrobenzofuran-5-carbonitrile hydrobromide (101 grams, 0.25 mole) prepared from 1-(3-Dimethylaminopropyl)-1-(4-fluorophenyl)-1,3-dihydro-5-isobenzofuranbromide, is suspended in water (500 ml) and toluene (500 ml). NaOH (60 ml, 5 N (aq)) is added and the mixture (pH>10) is stirred for 15 min. before the phases are separated. The organic phase is washed with water (2x100 ml) and filtered through a pad of filter help. The volatiles are removed *in vacuo* and the title compound is obtained as an oil. n-Heptane (400 ml) is added and the mixture is heated to 70 °C. On cooling, crystals form. The white crystals of the title compound are filtered off and dried at ambient temperature over night in vacuo. Yield: 75.4 grams (93%). DSC(onset, open capsule): 91.3-91.8 °C DSC (onset, closed capsule): 92.8 °C. Purity: (> 99.8 % (peak area)).
Anal. calcd. for C20H21N2F1O1; C, 74.04; H, 6.54; N, 8.64. Found C, 74.01; H, *6.49;* N, *8.59.* 1H-NMR (DMSO-d6, 500 MHz): 1.21 (1H, m), 1.29 (1H, m), 2.02 (6H, s), 2.09-2.23 (4 H, m), 5.15 (1H, d J=12.5 Hz), 5.22(1H, d J=12.5 Hz), 7.16 (2H, t J=8.5 Hz), 7.60 (2H, dt J=8.5 Hz J=1.2 Hz), 7.76 (1H, d J= 8.5 Hz), 7.79 (1H, d J=8.5 Hz), 7.80 (1H, s). 13C-NMR (DMSO-d6, 125 MHz): 21.8, 38.3, 45.0, 58.8, 71.0, 90.7, 110.5, 115.1 (d J=22 Hz), 118.8, 123.1, 125.1, 127.0 (d J=8 Hz), 132.0, 140.0 (d J=3 Hz), 140.5, 149.5, 161.3 (d J=245Hz).

### Example 2

### Preparation of the intermediate citalopram base

**a)** A crude mixture of Citalopram and sulphuric acid is made basic by adding NaOH and the citalopram base is extracted with toluene. The toluene is evaporated and the citalopram base obtained is dissolved in n-heptane at elevated temperature. The very pure free base of citalopram is precipitated by cooling.
**b)** A crude mixture of Citalopram and sulphuric acid is made basic by adding NaOH and the citalopram base is extracted with toluene. The toluene is evaporated and the citalopram base obtained is dissolved in methanol. The mixture is treated with activated carbon and filtrated and the solvent is evaporated. The purified free base is dissolved in n-heptane at elevated temperature. Then the very pure free base of citalopram is precipitated by cooling.
**c)** A crude mixture of Citalopram and sulphuric acid is made basic by adding NaOH and the citalopram base is extracted with toluene. The toluene phase is treated with silicagel, the toluene is evaporated and the citalopram base obtained is dissolved in n-heptane at elevated temperature. The very pure free base of citalopram is precipitated by cooling.
**d)** A crude mixture of Citalopram and sulphuric acid is made basic by adding NaOH and the citalopram base is extracted with toluene. The toluene phase is treated with silicagel, the toluene is evaporated and the citalopram base obtained is dissolved in methanol. The mixture is treated with activated carbon and filtrated and the solvent is evaporated. The purified free base is dissolved in n-heptanc at elevated temperature. Then the extremely pure free base of citalopram is precipitated by cooling.

## Claims

1. A process for the manufacture of a salt of citalopram **characterised in that** one or more impurities of the formula 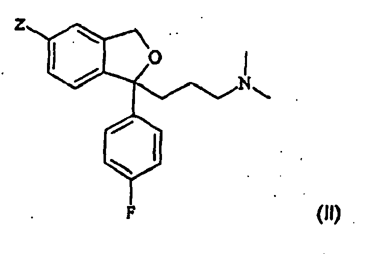 wherein Z is halogen, are removed from a crude mixture of citalopram or from a crude salt of citalopram, by precipitating citalopram base in crystalline form, and transferring said base into a salt thereof.

2. The process according to Claim 1 wherein the crude mixture of citalopram containing the compound of formula II as an impurity, is prepared by subjecting a compound of formula II to a cyanide exchange reaction with a cyanide source.

3. The process according to Claims 1 to 2 wherein the crude mixture of citalopram is subjected to initial purification before the base of citalopram is precipitated in crystalline form.

4. The process according to Claims 1 to 2 wherein the crude mixture of citalopram is subjected to initial purification before a crude salt is formed from said crude mixture.

5. The process according to Claims 1 to 4 wherein the base of citalopram is set free from a crude salt of citalopram by treatment with a base and optionally subjected to further purification before the base of citalopram is precipitated in crystalline form.

6. The process according to any of Claims 1 to 5 **characterised in that** the citalopram base is transferred into the hydrobromide or the hydrochloride salt of citalopram.

7. The process according to Claim 1, **characterised in that** the crude salt is a hydrobromide, hydrochloride, sulphate, oxalate, phosphate or nitrate salt, preferably the sulphate hydrobromide, or hydrochloride salt.

## Patentansprüche

1. Verfahren zur Herstellung eines Salzes von Citalopram, **dadurch gekennzeichnet, daß** eine oder mehrere Verunreinigungen der Formel 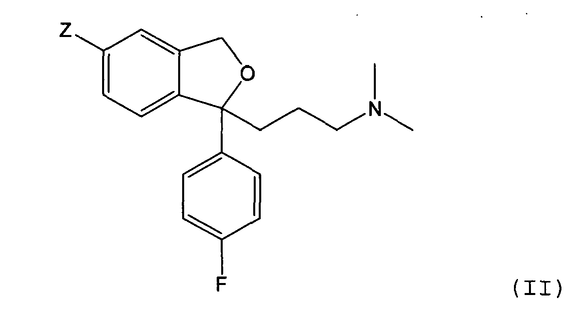 worin Z Halogen ist, aus einer rohen Mischung von Citalopram oder aus einem rohen Salz von Citalopram entfernt werden, indem Citalopram-Base in kristalliner Form ausgefällt und die Base in ein Salz davon überführt wird.

2. Verfahren gemäß Anspruch 1, worin die rohe Mischung von Citalopram, die die Verbindung der Formel (II) als eine Verunreinigung enthält, hergestellt wird durch Unterwerfen einer Verbindung der Formel (II) einer Cyanidaustauschreaktion mit einer Cyanidquelle.

3. Verfahren gemäß Ansprüchen 1 bis 2, worin die rohe Mischung von Citalopram einer anfänglichen Reinigung unterworfen wird, bevor die Base von Citalopram in kristalliner Form ausgefällt wird.

4. Verfahren gemäß Ansprüchen 1 bis 2, worin die rohe Mischung von Citalopram einer anfänglichen Reinigung unterworfen wird, bevor ein rohes Salz aus der rohen Mischung gebildet wird.

5. Verfahren gemäß Ansprüchen 1 bis 4, worin die Base von Citalopram aus einem rohen Salz von Citalopram durch Behandlung mit einer Base freigesetzt und ggf. einer weiteren Reinigung unterworfen wird, bevor die Base von Citalopram in kristalliner Form ausgefällt wird.

6. Verfahren gemäß einem der Ansprüche 1 bis 5, **dadurch gekennzeichnet, daß** die Citalopram-Base in das Hydrobromid- oder Hydrochloridsalz von Citalopram überführt wird.

7. Verfahren gemäß Anspruch 1, **dadurch gekennzeichnet, daß** das rohe Salz ein Hydrobromid-, Hydrochlorid-, Sulfat-, Oxalat-, Phosphat- oder Nitratsalz ist, bevorzugt das Sulfat-, Hydrobromid- oder Hydrochloridsalz.

## Revendications

1. Procédé pour préparer un sel de citalopram, **caractérisé en ce qu'**une ou plusieurs impuretés de formule dans laquelle Z est un halogéne 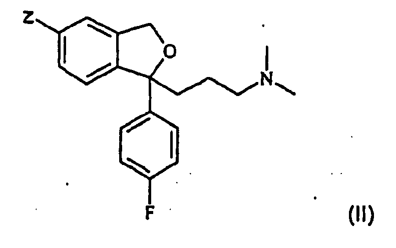 sont éliminées d'un mélange brut de citalopram ou d'un sel brut de citalopram, en précipitant du citalopram base sous forme cristalline, et en transformant cette base en un sel de celui-ci.

2. Procédé selon la revendication 1, dans lequel le mélange brut de citalopram contenant le composé de formule II comme impureté est préparé en soumettant un composé de formule II à une réaction d'échange de cyanure avec une source de cyanure.

3. Procédé selon l'une des revendications 1 à 2, dans lequel le mélange brut de citalopram est soumis à une purification initiale avant que le citalopram base ne soit précipité sous forme cristalline.

4. Procédé selon l'une des revendications 1 à 2, dans lequel le mélange brut de citalopram est soumis à une purification initiale avant qu'un sel brut ne soit formé à partir dudit mélange brut.

5. Procédé selon l'une des revendications 1 à 4, dans lequel le citalopram base est libéré d'un sel brut de citalopram par traitement avec une base et est soumis optionnellement à une autre purification avant que le citalopram base ne soit précipité sous forme cristalline.

6. Procédé selon l'une quelconque des revendications 1 à 5, **caractérisé en ce que** le citalopram base est transformé en le bromhydrate ou le sel chlorhydrate de citalopram.

7. Procédé selon la revendication 1, **caractérisé en ce que** le sel brut est un sel bromhydrate, chlorhydrate, sulfate, oxalate, phosphate ou nitrate, de préférence le sulfate, le bromhydrate ou le chlorhydrate.
